(19) Europäisches Patentamt
European Patent Office
Office européen des brevets

(11) Publication number: **0 168 680 B1**

# (12) EUROPEAN PATENT SPECIFICATION

(45) Date of publication of patent specification: **09.03.88**

(51) Int. Cl.⁴: **C 07 C 143/155**, C 07 C 143/675

(21) Application number: **85107751.1**

(22) Date of filing: **22.06.85**

## ERRATUM

| DIE TEXTSTELLE: / TEXT PUBLISHED: / LE PASSAGE SUIVANT: | (SEITE, SPALTE, ZEILE) (PAGE, COLUMN, LINE) (PAGE, COLONNE, LIGNE) | | LAUTET BERICHTIGT: / SHOULD READ: / DEVRAIT ETRE LU: |
|---|---|---|---|
| propionic anhydride and diketone | 7 | 56 | propionic anhydride and diketene. |

Tag der Entscheidung über die Berichtigung / Date of decision on rectification / Date de décision portant sur modification: 06.04.88

Ausgabe- und Veröffentlichungstag / Issue and publication date / Date d'edition et de publication: 01.06.88

Patbl.Nr / EPB no / Bull. no: 88/22

(19) Europäisches Patentamt
European Patent Office
Office européen des brevets

(11) Publication number: **0 168 680**
**B1**

(12) **EUROPEAN PATENT SPECIFICATION**

(45) Date of publication of patent specification: **09.03.88**

(51) Int. Cl.⁴: **C 07 C 143/155,**
C 07 C 143/675

(21) Application number: **85107751.1**

(22) Date of filing: **22.06.85**

(54) **Solid phase acylation of aminosulfonic acids.**

(30) Priority: **29.06.84 US 625941**
**24.04.85 US 725068**

(43) Date of publication of application:
**22.01.86 Bulletin 86/04**

(45) Publication of the grant of the patent:
**09.03.88 Bulletin 88/10**

(84) Designated Contracting States:
**CH DE FR GB IT LI**

(56) References cited:
**CH-A- 591 435**
**GB-A-2 128 189**

(73) Proprietor: **HOECHST CELANESE CORPORATION**
**Route 202-206 North**
**Somerville, N.J. 08876 (US)**

(72) Inventor: **Hazen, James R.**
**131 Wood Cove Drive**
**Coventry, R.I. 02816 (US)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80 (DE)**

**EP 0 168 680 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

This invention is in the field of preparing chemical intermediates and is directed to the acylation of aminosulfonic acids.

Non-gas phase bimolecular reactions generally do not readily occur unless conducted in the condensed phase, e.g. in a solvent or in the molten state. This condition, whether for homogeneous or heterogeneous reactions, is generally essential for the requisite intimate physical and chemical interactions of the reactants.

There are numerous prior art references to the acylation, in particular acetylation of various aminosulfonic acids in water. For example, sulfanilic acid has been acetylated with acetic anhydride in aqueous solution after prior neutralization with potassium carbonate (Chem. Ber. *58*, 2286). German Patent Specification No. 129,000 also teaches the acetylation of sulfanilic acid and a variety of other phenyl and naphthyl aminosulfonic acids in a similar fashion. A similar industrial process is described in the BIOS Final Report *1149*, p. 125, for the manufacture of acetyl sulfanilic acid. Chem. Ber. *46*, p. 755, and German Patent Specification No. 410,364 describe the acetylation of metanilic acid with acetic anhydride after neutralization in homogeneous aqueous solution, and J. Prakt, Chem. *80*, p. 201, describes the substantially equivalent acetylation of 1-naphthylamine-4-sulfonic acid. The resulting acetylated materials, however, are generally very soluble in water and isolation requires salting out with substantial quantities of an inorganic salt, such as sodium, potassium or ammonium sulfate or chloride. These procedures result in reduced yield because of solubility losses, and also result in contamination of the product with salts. Other costly methods, such as spray drying, or energy intensive methods, such as evaporating to dryness, have been utilized to separate the product from the aqueous solvent.

Other workers have avoided the problems associated with the aqueous acetylation of aminosulfonic acids by employing a non-aqueous solvent, such as acetic acid or pyridine. So, German Patent Specifications Nos. 69,555, 75,084 and 116,922 describe acetylations in acetic acid with sodium acetate as base while J. Org. Chem. *26*, 607, describes the use of acetic acid as solvent with pyridine as base. A number of works have utilized pyridine as solvent and base for the acetylation of aminosulfonic acids with acetic anhydride. Even the use of non-aqueous solvents, however, still requires the separation of the bulk of the solvent from the product by filtration.

Some workers have utilized the neutralized, isolated salt, such as an alkali metal or heavy metal salt, of the aminosulfonic acid as the starting material and conducted the acylation, especially acetylation, in excess acetic anhydride (Chem. Ber. *17*, 707; J. Prakt. Chem. *63*, 405; Chem. Ber. *39*, 1559). However, this has the disadvantage of introducing an additional step in the process, i.e., the isolation of the salt after base neutralization, in the case if the aminosulfonic acid is only available in its acid form. Furthermore, these publications teach that a solvent, such as water, alcohol or ether, or combinations of these, are required in the isolation of the acetylated products, again necessitating solvent removal.

The above prior art requires the solvent-wet product to be dried in yet another separate operation. Drying is essential, especially with water-wet products, since wet material is incompatible in the important chemical application of these materials, namely, conversion of the sulfonic acid group to the sulfochloride group with chlorosulfonic acid and/or thionyl chloride or similar reagent.

The invention has substantial advantages over prior art methods because the prior art involves doing the acylation usually in the homogeneous phase in a solvent, typically water. The present invention eliminates the solvent, salting out, filtration, and drying operation, and thereby substantially increases the process yield at a reduced cost. Importantly, in this age of environmental concern virtually all discharge of waste water or other liquid wastes is eliminated. The present invention directly produces a dry product in high purity and in very nearly quantitative yield. Furthermore, the process according to the invention is faster, more efficient and has higher space-time yields than prior technololgy.

In the present invention, the acylation occurs in the solid phase to produce a superior acylaminosulfonic acid at reduced reactants (acylation agent) and reduced costs. If the aminosulfonic acid starts from its acid form rather than from its neutral salt, the both discrete, sequential chemical reactions occur in the solid phase, i.e. the neutralization step of the sulfonic acid moiety with a base and the subsequent amine acylation, to produce a superior acylaminosulfonic acid at a reduced cost; both essential chemical steps occur virtually quantitatively in the solid phase.

This invention thus concerns a process for acylating an amino-sulfonic acid of the general formula (1)

$$(MO_3S)_m\text{—}A\text{—}(NH_2)_n \qquad (1)$$

wherein

M is the cation of the neutralizing agent, preferably an alkali metal or the equivalent of an alkaline earth metal, or is hydrogen,

A is a substituted or unsubstituted aliphatic, aromatic or heteroaromatic group,

m is the number 1, 2, 3 or 4, preferably 1 or 2, and

n is the number 1 or 2,

by means of an acylation agent to produce the corresponding N-acyl derivative or a sulfonic acid salt thereof of the general formula (2)

$$(MO_3S)_m—A—(NH—CO—R)_n \quad (2)$$

in which

R is an aliphatic radical or an aryl radical, preferably an alkyl group of 1 to 4 C-atoms or a phenyl group, and

M, A, m and n are defined as above,

with optional subsequent conversion of the salt obtained, such as of the alkali or alkaline earth metal salt, of the compound of formula (2) into the acylamino compound according to formula (2) in which M is hydrogen, by means of an acid such as a mineral acid, for example, hydrochloric acid or sulfuric acid, in the conventional manner of converting a salt of a sulfonic acid into the acid form, which process is characterized in that the required neutralization if M is hydrogen in the compound of formula (1), and the (subsequent) acylation reaction is carried out in the solid, semi-solid or dough-like state and in the absence of any added solvent or other vehicle to facilitate the reaction under the action of intensive mixing the reaction components and agitation of the solid phase.

In the formula (1), M is preferably a hydrogen atom. The formula member A is preferably an unsubstituted or substituted benzene or naphthalene group. The moiety A includes in particular alkane, aralkane, benzene, naphthalene and heteroaromatic groups, which may contain substituents, such as halogen, alkyl, alkoxy, hydroxy, sulfo, nitro and/or acylamino groups. Alkyl and alkoxy are preferably of 1 to 4 C-atoms; aryl is preferably phenyl and naphthyl, optionally substituted, such as by alkoxy, alkyl, halogen, nitro, sulfo, carboxy, sulfamoyl and/or carbamoyl. The process is especially useful in the production of acetylated aminoaryl sulfonic acids, a number of which are important dyestuff precursors.

The reaction mass consists only of the reactants, i.e. the starting dry or nearly dry aminosulfonic acid, or its neutral salt, the acylating agent and some organic or inorganic compound capable of neutralizing the sulfonic acid moiety if necessary, because little or no acylation of the amino group occurs if the aminosulfonic acid is not neutralized. Suitable neutralizing compounds or bases include the alkali or other metal carboxylates, carbonates, hydroxides, alkoxides or similar oxygen bases as well as nitrogeneous bases such as ammonia and amines. Preferred neutralization agents include hydroxides, acetates and carbonates of the alkali and alkaline earth metals. Most preferred are the hydroxides, acetates and carbonates of sodium, potassium, lithium and calcium. The foregoing examples of suitable neutralizing compounds are illustrative and it will be readily apparent to one skilled in the art that there exists a large number of alternative inorganic and organic neutralizing compounds.

Examples of acylating agents include the carboxylic acid anhydrides of aliphatic and aromatic carboxylic acids, such as preferably acetic and propionic anhydrides, and other similar reactive acylating agents, such as diketene.

The present invention concerns in particular a process for the preparation of an acylamino sulfonic acid of the general formula (2)

$$(MO_3S)_m—A—(NH—CO—R)_n \quad (2)$$

in which M is hydrogen or an alkali metal or an equivalent of an alkaline earth metal, R is an alkyl of 1 to 4 carbon atoms or is phenyl, A is a benzene or naphthalene ring which is additionally not substituted or additionally substituted by substituents selected from the group consisting of halogen, such as fluorine, bromine and preferably chlorine, alkyl of 1 to 4 carbon atoms, alkoxy of 1 to 4 carbon atoms, hydroxy, nitro, alkanoylamino of from 2 to 5 carbon atoms and benzoylamino, or is an alkylene group of 2 to 6 C-atoms, m is the integer 1, 2, 3 or 4, and n is the integer 1 or 2,

which is characterized in that a compound of the general formula (1)

$$(MO_3S)_m—A—(NH_2)_n \quad (1)$$

in which M, A, m and n have the meanings mentioned above, M representing preferably a hydrogen atom, is reacted with an acylating agent according to the general formula (3a) or (3b)

$$R—CO—X \quad (3a)$$

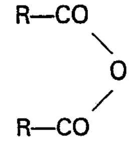

(3b)

in which R has the meaning mentioned above and X represents the hydroxy group or a halogen atom, such as a bromine and preferably chlorine atom, in an intensively operating mixing device, preferably in a machine working with a kneading effect, if necessary in the presence of an acid-binding agent, such as an alkaline or alkaline earth-alkaline agent to neutralize the acid compound of formula (1) if M is hydrogen,

however, in the absence of any solvent or of any other auxiliary agent which facilitates or advances the acylation reaction, and the alkali or alkaline earth metal salt of the compound of formula (2) obtained is optionally converted into the acylamino compound according to formula (2), in which M is hydrogen, by means of an acid.

In the above formulae (1) and (2), the radical A represents preferably an alkane radical of 2 to 6 C-atoms and in particular a benzene ring which may additionally be substituted by 1 or 2, preferably 1, substituent(s) selected from the group consisting of alkyl of 1 to 4 C-atoms, alkoxy of 1 to 4 C-atoms and chlorine, or in particular a naphthalene ring optionally additionally substituted by hydroxy, and m is 1 or 2, and n is 1.

Those aminosulfonic acids of the formula (1) are, for example, 2-aminobenzenesulfonic acid, 3-aminobenzenesulfonic acid, 4-aminobenzenesulfonic acid, 3-methyl-4-aminobenzenesulfonic acid, 2,4-diaminobenzenesulfonic acid, 1-amino-ethane-2-sulfonic acid, 1-amino-naphthalene-4-sulfonic acid, 1-amino-naphthalene-5-sulfonic acid, 1-amino-naphthalene-8-sulfonic acid, 2-amino-naphthalene-1-sulfonic acid, 2-amino-naphthalene-5-sulfonic acid, 2-amino-naphthalene-6-sulfonic acid and 2-amino-naphthalene-8-sulfonic acid, 1-amino-8-naphthol-3,6- and -4,6-disulfonic acid.

The reaction is conducted by mixing the reactants in equipment having the capability required for the mixing of moist or dry solids or otherwise viscous, heavy or tacky materials which may pass through a plastic or dough-like state. Examples of such suitable apparatus are double arm kneader (continuous or batch), ribbon blender, pan dryer, paddle dryer, rotary or similar turbulent drying equipment.

The physical state of the reaction mass during the course of the reaction is dependent on the choice of starting materials and reaction time, and can be a more or less free flowing powder, a moist solid or a dough-like mass or a combination of these states. The starting material and a molar excess of base in the range of up to 100% excess, preferably about 5% excess (a small amount of the base may also be used if the starting compound (1) is a neutral salt), are pre-mixed in the reactor, e.g., a kneader, and then treated in a controlled manner with a molar excess of acylating agent in the range of up 100%, preferably 25 to 50%, most preferred about 50% excess. The reaction temperature is usually not a critical parameter and the reaction is usually done without external temperature control but such control may be applied where necessary. Depending on the reactants, the reaction may be readily completed in the temperature range from about ambient to about 100°C, preferably from about 30°C to about 80°C. The reaction is typically accompanied by a moderate, brief exotherm which peaks at about 40—50°C and is rapid, being generally kinetically complete in less than an hour.

Typically the reactants will form a dough-like mass during or shortly after the addition of acylating agent which may start to revert back to a solid powder form towards the end of the reaction. The formation of the reaction products powdery form can be facilitated by removing the volatile material (e.g. water, excess acylating agent, or by-products derived therefrom) from the system by heating the mixture or maintaining it under reduced pressure, or both. The removal of volatile matter is desirable not only to facilitate the discharge of the product in powder form from the reactor but also to free it of odiferous or noxious volatiles or materials, such as water, which are chemically incompatible in certain further chemical processing of the product. Typically, by maintaining the reaction mass under reduced pressure with external heating for one to two hours, the dry acylated product can be recovered in nearly quantitative yield in high purity. Alternatively, the volatile materials may be removed in a stream of air or nitrogen or other suitable gas. The conversion of amine to acylated amine is generally greater than 98%.

If amino-aryl sulfonic acids of formula (1), which contain a phenolic or naphtholic hydroxy group as a substituent, are used, some acylation of these hydroxy groups, too, may occur. However, the acylated hydroxy group is deacylated again during the above-mentioned drying step.

The process according to the invention of acylating aminosulfonic acids, in particular with acetic anhydride, has substantial advantages over prior art methods. Prior art technology involves doing the acylation reactions in a solvent, typically water, as a vehicle for the required base neutralization and subsequent acylation. The resulting acylated materials, however, are very soluble in aqueous solvents, requiring the salting out of the product with substantial quantities of inorganic salts, such as sodium or ammonium sulfate, in order to isolate it by filtration. Such salting out operations inevitably result in loss of yield via solubility losses and contamination of the product with salts. Furthermore, certain acylated aminosulfonic acids are so water-soluble that they cannot readily be salted out with inorganic salts and are inconvenient difficult to isolate. In cases such as these, as for example with 1-acetaminonaphthalene-5-sulfonic acid and 2-acetamino-naphthalene-5-sulfonic acid, the product can only be precipitated from aqueous solution by acidifying to below pH 1 with a strong mineral acid, such as hydrochloric acid. This particular technique is undesirable relative to the present invention for two additional reasons. The first is that such products present special safety and handling difficulties due to the fact that they are highly acidic. The second is that such acidic materials present special difficulties during drying due to the corrosive nature of the mineral acids and their vapors. Such drying requires specialized corrosion-resistant drying equipment. The solid phase technique is uniquely advantageous not only in its simplicity of operation but also by its universality of application to virtually all aminosulfonic compounds regardless of variations in chemical structure, water solubility and other physical and chemical properties.

Yet another and unanticipated advantage of the solid phase method relative to the aqueous methods is found in those products which are usually isolated from aqueous solutions by salting out with ammonium sulfate. Two examples of such products isolated as their ammonium salts are 2-acetamino-naphthalene-6-sulfonic acid and 2-acetaminonaphthalene-8-sulfonic acid. These products if prepared via the solid phase method as their sodium salts give distinctly higher yields than the corresponding ammonium salts in a desired subsequent reaction to convert them to their corresponding sulfonyl chlorides. In particular, the 2-acetaminonaphthalene-6-sulfonic acid via the solid phase technique can be converted to the sulfochloride consistently in an 8 to 9% higher yield (95% vs. 86% of theory) than the product via the aqueous ammonium sulfate salting out technique. Similar results are obtained with the conversions of 2-acetamino-naphthalene-8-sulfonic acid salts to the sulfochloride. These results are demonstratably due to the yield-lowering effect of the ammonium ion versus the sodium ion in the chlorosulfonation.

Previously other cumbersome methods such as spray-drying for separating the product from the solvent have been utilized. Even the use of non-aqueous solvents, such as acetic acid, still entails the necessity of separating the product from the bulk of the solvent vehicle by filtration. These prior art technologies, moreover, still require that the isolated solvent-wet products be dried in a separate operation. Drying is essential, particularly with water-wet products, since wet material is incompatible in most chemical applications of these products, namely the conversion of the sulfonic acid to its sulfochloride with chlorosulfonic acid and/or thionyl chloride.

The present invention, by eliminating the solvent, eliminates the salting out, filtration and subsequent drying steps, and thereby also eliminates yield losses and, importantly, virtually all discharge of waste water. Not only are large waste water discharges eliminated, but also the solid phase technique readily allows the recovery of the volatile organic by-product from the acylation by simple methods. For example, in the case of acetic anhydride as the acylating agent, the valuable by-product acetic acid can be recovered and reclaimed nearly quantitatively simply by condensing its vapors during the drying operation. Such a recovery is not feasible in aqueous acetylations (or other acylations), and all the by-product acetic acid must be discarded in the waste water. The present invention directly produces a dry product in high purity and in nearly quantitative yield. Furthermore, the process described herein is faster and has higher spacetime yields than the existing technology, and provides a very simple and general method which is universally applicable to all aminosulfonic acids and gives products in the most suitable physical and chemical form for subsequent chemical conversions.

The Examples which follow serve to illustrate the invention. The parts are parts by weight, and the percentage data are percentages by weight, unless otherwise noted. Parts by weight relate to parts by volume as the kilogram relates to the liter.

## Example 1

40 Parts of 2-naphthylamine-6-sulfonic acid are charged into a double-arm kneader with a sigma blade configuration having a capacity of 150 parts by volume, followed by 150 parts of a 50% aqueous sodium hydroxide with mixing. After mixing for 10—15 minutes, 27 parts of acetic anhydride are added over about five minutes. The reaction mixture forms a soft dough-like mass and the temperature reaches a maximum of about 40—45°C within 10—15 minutes. After about 30 minutes the reaction mass is heated externally with steam under reduced pressure to remove water, acetic acid, and excess acetic anhydride. After one to two hours the dry, powdery sodium salt of the 2-acetylamino-naphthalene-6-sulfonic acid is discharged from the kneader. The reaction yield is 95 percent, and liquid chromatographic analysis indicated a purity of 93% acetylated product which contains a small amount (1.5%) of the unconverted starting sulfonic acid.

## Example 2

37.5 Parts of metanilic acid (3-aminobenzenesulfonic acid) and 18.7 parts of sodium acetate are charged into the kneader, and 32 parts of acetic anhydride are added to the reaction mixture, and the procedure described in Example 1 is followed. The powdery reaction mass is mixed for one hour and then dried at 85°C in a stream of air to give a 95% yield of 3-acetylamino-benzenesulfonic acid sodium salt. Liquid chromatography and titration analysis indicated a purity of greater than 97% and the presence of about 0.5% metanilic acid.

## Example 3

Metanilic acid in an amount of 37.5 parts and 9.1 parts of sodium hydroxide beads are mixed in a kneader for about 30 minutes and then 32 parts of acetic anhydride are added over a period of 7—8 minutes. During the anhydride addition, the reaction mixture forms a soft dough-like mass. After about one-half hour, the dough-like mass (temperature ca. 40°C) starts to revert to a moist solid. After one hour, external steam heating is applied and the mixture held under vacuum. After 1.5 hours the dry powder is discharged to yield 50.9 parts of white powder, having a 97.9% pure acet-metanilic acid sodium salt containing 0.1% metanilic acid (96.9% of the theoretical yield).

## Examples 4 to 10

The results from other representative aminosulfonic acids which have been similarly acetylated as described in the above Examples 1 to 3, are presented in the Table below.

| Ex. | Starting Compound (2) | Base (mole ratio) | Acetic anhydride (mole ratio) | Yield |
|---|---|---|---|---|
| 4 | Sulfanilic acid | 50% NaOH, 1.03 | 1.48 | 96.7% |
| 5 | 4-Amino-m-toluene | NaOH, 1.04 | 1.50 | 90.3% |
| 6 | 2-Amino-naphthalene-1-sulfonic acid | NaOAc, 1.03 | 1.47 | 95.5% |
| 7 | 1-Amino-naphthalene-5-sulfonic acid | 50% NaOH, 1.05 | 1.47 | 93.8% |
| 8 | 2-Amino-naphthalene-8-sulfonic acid | NaOH, 1.08 | 1.72 | 96.0% |
| 9 | 1,3-Phenylenediamine-4-sulfonic acid | NaOAc, 1.05 | 2.94 | 90.0% |
| 10 | 1-Amino-ethane-2-sulfonic acid | NaOH, 1.05 | 1.50 | 97.0% |

Example 11

1-Acetylamino-8-naphthol-3,6-disulfonic acid was prepared from 1-amino-8-naphthol-3,6-disulfonic acid as follows: 60 parts of a product containing 87.6% of 1-amino-8-naphthol-3,6-disulfonic acid as the monosodium salt and 10.6% of water (as crystal water) were charged into a kneader; 15 parts of sodium acetate were added. After the solids were mixed for a brief period, 24 parts of acetic anhydride were added gradually with continued mixing. When the addition was complete, the reactants were heated by passing steam at a temperature of about 100°C through the jacket of the kneader for about 1 hour. The kneading mass was then dried by continued heating in a stream of air, then cooled, and the powder discharged from the kneader to give 61 parts of the sodium salt of the 1-acetylamino-8-naphthol-3,6-disulfonic acid which by liquid chromatography analysis contains about 2.5% of the starting aminonaphthol-disulfonic acid and about 2.5% of 1-acetylamino-8-acetyloxy-naphthalene-3,6-disulfonic acid.

**Claims**

1. A process for producing an acylamino sulfonic acid of the general formula (2)

$$(MO_3S)_m—A—(NH—CO—R)_n \qquad (2)$$

in which M is the cation of the neutralizing agent, preferably an alkali metal or the equivalent of an alkaline earth metal, R is an aliphatic radical or an aryl radical, A is a substituted or unsubstituted aliphatic, aromatic or heteroaromatic group, m is the number 1, 2, 3 or 4, and n is the number 1 or 2, which process is characterized by carrying out the step of neutralizing the acid compound of the general formula (1a)

$$(HO_3S)_m—A—(NH_2)_n \qquad (1a)$$

wherein A, m and n are defined as above, by means of a neutralizing agent and the subsequent step of acylating this neutralized aminosulfonic acid in the solid, semi-solid or dough-like state and in the absence of any added solvent or other vehicle to facilitate the reaction, under the action of intensive mixing the reaction components and agitation of the solid phase.

2. A modification of the process of claim 1, in which the starting compound is a neutral salt according to the general formula (1b)

$$(MO_3S)_m—A—(NH_2)_n \qquad (1b)$$

in which M, A, m and n are defined as in claim 1, which process is characterized in carrying out the acylation reaction in the solid, semi-solid or dough-like state and optionally in the presence of a small amount of a basic compound, however, in the absence of any solvent or other auxiliary agent to facilitate or advance the reaction, under the action of intensive mixing the reaction components and agitation of the solid phase.

3. A process according to claim 1 or 2, characterized in that the neutral salt of formula (2) obtained is converted into the acid compound of formula (2) in which M is herein a hydrogen atom, by means of an acid.

4. The process according to Claim 1, 2 or 3, wherein said neutralized acyl-aminosulfonic acid is dried by vaporizing volatile components from said reaction mass.

5. The process according to Claim 4 wherein said vaporization is conducted under reduced pressure.

6. The process according to Claim 1 or 2, wherein said neutralizing agent is selected from the group consisting metal carboxylates, carbonates, hydroxides, alkoxides, and oxides.

7. The process according to Claim 6, wherein said metal is selected from the group consisting of sodium, lithium, potassium and calcium.

8. The process according to Claim 1, 2 or 3, wherein A is an alkane, arylalkane, substituted or unsubstituted benzene, naphthalene or heteroaromatic group.

9. The process according to Claim 8 wherein the substituent(s) in said substituted benzene or naphthalene are independently selected from the group consisting of halogen, alkyl, alkoxy, hydroxy, nitro and acylamino.

10. A process according to one of Claims 1 to 7 for the preparation of an acylamino sulfonic acid of the general formula (2)

$$(MO_3S)_m—A—(NH—CO—R)_n \qquad (2)$$

in which M is hydrogen or an alkali metal or an equivalent of an alkaline earth metal, R is an alkyl of 1 to 4 carbon atoms or is phenyl, A is a benzene or naphthalene ring which is additionally not substituted or additionally substituted by substituents selected from the group consisting of halogen, such as fluorine, bromine and preferably chlorine, alkyl of 1 to 4 carbon atoms, alkoxy of 1 to 4 carbon atoms, hydroxy, nitro, alkanoylamino of from 2 to 5 carbon atoms and benzoylamino, or is an alkylene group of 2 to 6 C-atoms, m is the integer 1, 2, 3 or 4, and n is the integer 1 or 2, characterized in that a compound of the general formula (1)

$$(MO_3S)_m—A—(NH_2)_n \qquad (1)$$

in which M, A, m and n have the meanings mentioned above, M representing preferably a hydrogen atom, is reacted with an acylating agent according to the general formula (3a) or (3b)

$$R—CO—X \qquad (3a)$$

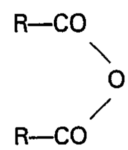

(3b)

in which R has the meaning mentioned above and X represents the hydroxy group or a halogen atom, such as a bromine and preferably chlorine atom, in an intensively operating mixing device, preferably in a machine working with a kneading effect, if necessary in the presence of an acid-binding agent, such as an alkaline or alkaline earth-alkaline agent to neutralize the acid compound of formula (1) if M is hydrogen, however, in the absence of any solvent or of any other auxiliary agent which facilitates or advances the acylation reaction, and the alkali or alkaline earth metal salt of the compound of formula (2) obtained is optionally converted into the acylamino compound according to formula (2), in which M is hydrogen, by means of an acid.

11. A process according to claim 1, 2, 3 or 10, in which the formula moiety A represents an alkane radical of 2 to 6 C-atoms, a benzene ring which may additionally be substituted by 1 or 2 substituents selected from the group consisting of alkyl of 1 to 4 C-atoms, alkoxy of 1 to 4 C-atoms and chlorine, or a naphthalene ring, optionally substituted additionally by a hydroxy group, m is 1 or 2, and n is 1.

12. The process according to Claim 1, 2, 3 or 10, wherein A is an unsubstituted benzene or naphthalene ring and m is 1 or 2 and n is 1.

13. The process according to Claim 1, 2, 3 or 10, wherein the acylating agent is used in an amount of about 25—50 percent molar excess.

14. The process according to Claim 1, 2, 3 or 10, wherein the acylating agent is selected from the group consisting of acetic anhydride, propionic anhydride and diketone.

15. A process according to any one of claims 1, 2 or 4 to 14, characterized by that the process is carried out in a kneader.

**Patentansprüche**

1. Verfahren zur Herstellung einer Acylaminosulfonsäure der allgemeinen Formel (2)

$$(MO_3S)_m—A—(NH—CO—R)_n \qquad (2)$$

worin M das Kation des Neutralisationsmittels, vorzugsweise ein Alkalimetall oder das Äquivalent eines Erdalkalimetalls, R einen aliphatischen Rest oder einen Arylrest, A eine gegebenenfalls substituierte aliphatische, aromatische oder heteroaromatische Gruppe, m die Zahl 1, 2, 3 oder 4 und n die Zahl 1 oder 2 darstellen, dadurch gekennzeichnet, daß man die Stufe der Neutralisierung der sauren Verbindung der allgemeinen Formel (1a)

$$(HO_3S)_m—A—(NH_2)_n \qquad (1a)$$

worin A, m und n die obigen Bedeutungen haben, mittels einem Neutralisationsmittel und die anschließende Stufe der Acylierung dieser neutralisierten Aminosulfonsäure jeweils im festen, halbfesten oder teigartigen Zustand und in Abwesentheit jeglichen zugesetzten Lösungsmittels oder sonstigen Trägers zur Erleichterung der Reaktion unter intensiver Vermischung der Reaktionskomponenten und mechanischer Behandlung der festen Phase durchführt.

2. Abänderung des Verfahrens nach Anspruch 1, worin die Ausgangsverbindung ein neutrales Salz gemäß der allgemeinen Formel (1b)

$$(MO_3S)_m—A—(NH_2)_n \qquad (1b)$$

ist, worin M, A, m und n die in Anspruch 1 angegebenen Bedeutungen haben, dadurch gekennzeichnet, daß man die Acylierungsreaktion im festen, halbfesten oder teigähnlichen Zustand und gegebenenfalls in Gegenwert einer geringen Menge einer basischen Verbindung, jedoch in Abwesenheit irgendeines Lösungs- oder sonstigen Hilfsmittels zur Erleichterung oder Förderung der Reaktion unter intensiver Vermischung der Rekationskomponenten und mechanischer Behandlung der festen Phase durchführt.

3. Verfahren nach Anspruch 1 oder 2, dadurch gekennzeichnet, daß das erhaltene neutrale Salz der Formel (2) mittels einer Säure in die saure Verbindung der Formel (2), worin M für ein Wasserstoffatom steht, überführt wird.

4. Verfahren nach Anspruch 1, 2 oder 3, worin die neutralisierte Acylaminosulfonsäure durch Verdampfen flüchtiger Komponenten aus dieser Reaktionsmasse getrocknet wird.

5. Verfahren nach Anspruch 4, worin die Verdampfung bei vermindertem Druck erfolgt.

6. Verfahren nach Anspruch 1 oder 2, worin das Neutralisationsmittel aus der Gruppe der Metallcarboxylate, -carbonate, -hydroxide, -alkoholate und -oxide ausgewählt ist.

7. Verfahren nach Anspruch 6, worin dieses Metall aus der Natrium, Lithium, Kalium und Calcium umfassenden Gruppe ausgewählt ist.

8. Verfahren nach Anspruch 1, 2 oder 3, worin A eine Alkan-, Arylalkan- oder eine gegebenenfalls substituierte Benzol-, Naphthalin- oder heteroaromatische Gruppe darstellt.

9. Verfahren nach Anspruch 8, worin der bzw. die Substituent(en) in jenem substituierten Benzol oder Naphthalin unabhängig voneinander aus der Gruppe von Substituienten, bestehend aus Halogen, Alkyl, Alkoxy, Hydroxy, Nitro und Acylamino, ausgewählt sind.

10. Verfahren nach einem der Ansprüche 1 bis 7 zur Herstellung einer Acylaminosulfonsäure der allgemeinen Formel (2)

$$(MO_3S)_m—A—(NH—CO—R)_n \qquad (2)$$

worin M Wasserstoff oder ein Alkalimetall oder ein Äquivalent eines Erdalkalimetalls, R Alkyl mit 1 bis 4 Kohlenstoffatomen oder Phenyl, A einen Benzol- oder Naphthalinring mit gegebenenfalls zusätzlichen Substituenten, die aus der Halogen, wie Fluor, Brom und vorzugsweise Chlor, Alkyl mit 1 bis 4 Kohlenstoffatomen, Alkoxy mit 1 bis 4 Kohlenstoffatomen, Hydroxy, Nitro, Alkanoylamino mit 2 bis 5 Kohlenstoffatomen und Benzoylamino umfassenden Gruppe ausgewählt sind, oder eine Alkylengruppe mit 2 bis 6 Kohlenstoffatomen, m die ganze Zahl 1, 2, 3 oder 4 und n die ganze Zahl 1 oder 2 darstellen, dadurch gekennzeichnet, daß man eine Verbindung der allgemeinen Formel (1)

$$(MO_3S)_m—A—(NH_2)_n \qquad (1)$$

worin M, A, m und n die obigen Bedeutungen haben, wobei M vorzugsweise für eine Wasserstoffatom steht, mit einem Acylierungsmittel gemäß der allgemeinen Formel (3a) oder (3b)

$$R—CO—X \qquad (3a)$$

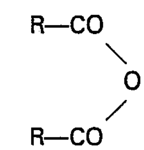

(3b)

worin R die oben angegebene Bedeutung hat und X für die Hydroxygruppe oder ein Halogenatom, wie ein

Brom- und vorzugsweise Chloratom, steht, in einer Intensivmischvorrichtung, bevorzugt in einer Maschine mit Knetwirkung, nötigenfalls in Gegenwart eines säurebindenden Mittels, wie einem alkalischen oder erdalkalischen Mittle, zur Neutralisation der sauren Verbindung der Formel (1), falls M für Wasserstoff steht, jedoch in Abwesentheit irgendeines Lösungsmittels oder sonstigen Hilfsmittels, das die Acylierungsreaktion erleichtert oder fördert, umsetzt und das erhaltene Alkali- oder Erdalkalisalz der Verbindung der Formel (2) gegebenenfalls mittels einer Säure in die Acylaminoverbindung gemäß der Formel (2), worin M für Wasserstoff steht, überführt.

11. Verfahren nach Anspruch 1, 2, 3 oder 10, worin der Formelteil A einen Alkanrest mit 2 bis 6 Kohlenstoffatomen, einen gegebenenfalls zusätzlich durch 1 oder 2 Substituenten aus der Gruppe Alkyl mit 1 bis 4 Kohlenstoffatomen, Alkoxy mit 1 bis 4 Kohlenstoffatomen und Chlor substituierten Benzolring oder einen gegebenenfalls zusätzlich durch eine Hydroxylgruppe substituierten Naphthalinring, m 1 oder 2 und n die Zahl 1 bedeuten.

12. Verfahren nach Anspruch 1, 2, 3 oder 10, worin A einen unsubstituierten Benzol- oder Naphthalinring, m 1 oder 2 und n die Zahl 1 bedeuten.

13. Verfahren nach Anspruch 1, 2, 3 oder 10, worin das Acylierungsmittel in einem etwa 25—50%igen molaren Überschuß eingesetzt wird.

14. Verfahren nach Anspruch 1, 2, 3 oder 10, worin das Acylierungsmittel Essigsäureanhydrid, Propionsäuranhydrid oder Diketen ist.

15. Verfahren nach einem der Ansprüche 1, 2 oder 4 bis 14, dadurch gekennzeichnet, daß man das Verfahren in einem Kneter durchführt.

**Revendications**

1. Un procédé de production d'acide acylamino-sulfoniques de formule générale (2) ci-dessous:

$$(MO_3S)_m\text{—}A\text{—}(NH\text{—}CO\text{—}R)_n \qquad (2)$$

(dans laquelle M représente le cation de l'agent neutralisant, de préférence un métal alcalin ou l'équivalent d'un métal alcalino-terreux, R est un radical aliphatique ou arylique, A un radical aliphatique, aromatique ou hétéroaromatique avec ou sans substituants, m le nombre 1, 2, 3 ou 4 et n le nombre 1 ou 2), procédé caractérisé en ce que l'on effectue la neutralisation d'un acide de formule générale (1a):

$$(HO_3S)_m\text{—}A\text{—}(NH_2)_n \qquad (1a)$$

A, m et n ayant les significations ci-dessus, avec un agent de neutralisation, ainsi que l'acylation de l'acide aminosulfonique ainsi neutralisé, à l'état solide, semi-solide ou pâteux et sans aucun solvant ou autre véhicule pour faciliter la réaction, avec un effet de mélange intense des réactifs par agitation de la phase solide.

2. Une modification du procédé selon la revendication 1 dans laquelle on part d'un sel neutre de formule générale (1b):

$$(MO_3S)_m\text{—}A\text{—}(NH_2)_n \qquad (1b)$$

M, A, m et n ayant les mêmes significations que dans la revendication 1, procédé caractérisé en ce que l'on effectue la réaction d'acylation à l'état solide, semi-solide ou pâteux et le cas échéant en présence d'une petite proportion d'un composé basique, mais sans aucun solvant ou autre agent auxiliaire pour faciliter ou faire avancer la réaction, avec un effet de mélange intense des réactifs par agitation de la phase solide.

3. Un procédé selon la revendication 1 ou 2 caractérisé en ce que le sel neutre de formule (2) obtenu est transformé en acide de formule (2), c'est-à-dire un composé dans lequel M est un atome d'hydrogène, au moyen d'un acide.

4. Un procédé selon la revendication 1, 2 ou 3 dans lequel on sèche l'acide acyl-aminosulfonique neutralisé en évaporant les composants volatils de la masse réactionnelle.

5. Le procédé selon la revendication 4 dans lequel on effectue l'évaporation sous pression réduite.

6. Le procédé selon la revendication 1 ou 2 dans lequel l'agent de neutralisation est un carboxylate, un carbonate, un hydroxyde, un alcoxyde ou un oxyde de métal.

7. Le procédé selon la revendication 6 dans lequel le métal est le sodium, le lithium, le potassium ou le calcium.

8. Le procédé selon la revendication 1, 2 ou 3 dans lequel, dans les formules indiquées, A est un radical d'alcane, d'arylalcane, de benzène ou de naphtalène, substitués ou non, ou un groupe hétéroaromatique, substitué ou non.

9. Le procédé selon la revendication 8 dans lequel le ou les substituants du benzéne ou du naphtalène sont choisis, indépendamment les uns des autres, parmi les halogènes, des alkyles, dès alcoxy, les groupes hydroxy et nitro et acylamino.

10. Un procédé selon l'une quelconque des revendications 1 à 7 pour la préparation d'acides acylaminosulfoniques de formule générale (2):

$$(MO_3S)_m\text{—}A\text{—}(NH\text{—}CO\text{—}R)_n \qquad (2)$$

(dans laquelle M désignel l'hydrogène ou bien un métal alcalin ou l'équivalent d'un métal alcalino-terreux, R un alkyle en $C_1$—$C_4$ ou un phényle, A un radical de benzène ou de naphtalène sans substituants ou avec des substituants choisis parmi des halogènes tels que le fluor, le brome et de préférence le chlore, des alkyles et alcoxy en $C_1$—$C_4$, les groupes hydroxy et nitro, et alcanoylamino en $C_2$—$C_5$ et benzoylamino, ou encore A peut être un alkylène en $C_2$—$C_6$, m est le nombre 1, 2, 3 ou 4 et n le nombre 1 ou 2), procédé caractérisé en ce que l'on fait réagir un composé de formule générale (1):

$$(MO_3S)_m\text{—}A\text{—}(NH_2)_n \qquad (1)$$

M, A, m et n ayant les significations précédentes mais M étant de préférence un atome d'hydrogène, avec un agent d'acylation de formule générale (3a) ou (3b):

$$R\text{—}CO\text{—}X \qquad (3a)$$

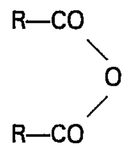
(3b)

R ayant la signification précédemment indiquée et X représentant un hydroxyle ou un atome d'halogène, tel que le brome et de préférence le chlore, dans un appareil à effet de mélange intense, de préférence ayant un effet de malaxage, si cela est nécessaire en présence d'un agent liant les acides tel qu'un agent alcalin ou alcalino-terreux pour neutraliser l'acide de formule (1) si M est l'hydrogène, mais sans aucun solvant ou autre agent auxiliaire facilitant ou faisant avancer la réaction d'acylation, et le cas échéant on transforme le sel de métal alcalin ou alcalinoterreux du composé de formule (2) ainsi obtenu en composé acylaminé de formule (2) dans lequel M est l'hydrogène, par réaction avec un acide.

11. Un procédé selon la revendication 1, 2, 3 ou 10 dans lequel, dans les formules indiquées, A représente un radical d'alcane en $C_2$ à $C_6$, un radical de benzène pouvant avoir 1 ou 2 substituants choisis parmi des alkyles et des alcoxy en $C_1$ à $C_4$ et le chlore, ou un radical de naphtalène pouvant porter un groupe hydroxy, m est le nombre 1 ou 2 et n le nombre 1.

12. Le procédé selon la revendication 1, 2, 3 ou 10 dans lequel A est un radical de benzène ou de naphtalène sans substituants, m est le nombre 1 ou 2 et n le nombre 1.

13. Le procédé selon la revendication 1, 2, 3 ou 10 dans lequel l'agent d'acylation est ajouté en un excès molaire d'environ 25 à 50%.

14. Le procédé selon la revendication 1, 2, 3 ou 10 dans lequel l'agent d'acylation est choisi parmi l'anhydride acétique, l'anhydride propionique et le dicétène.

15. Un procédé selon l'une quelconque des revendications 1, 2, et 4 à 10, caractérisé en ce que l'on opère dans un malaxeur.